# EUROPEAN PATENT APPLICATION

(11) **EP 3 059 312 A1**
(43) Date of publication of application: **24.08.2016**
(21) Application number: 15156019.0
(22) Date of filing: 20.02.2015
(51) Int. Cl.: C12N 15/10, C12Q 1/68

(54) **Nucleic acid extraction method**

(71) Applicant: QIAGEN GmbH, 40724 Hilden (DE)
(72) Inventor: Fakih, Sarah, 40724 Hilden (DE); Himmelreich, Ralf, 55131 Mainz (DE); Cornelius, Petra, 51069 Köln (DE); Engel, Holger, 40723 Hilden (DE)
(74) Representative: Roth, Carla

(57) **Abstract**

The present invention provides a method for extracting a nucleic acid from a sample and depleting inhibitors contained in the sample, comprising:
a) preparing an extraction mixture by contacting the sample with
i) at least one cationic detergent capable of forming a precipitate with inhibitors contained in the sample,
ii) at least one salt, wherein the salt is contained in the extraction mixture in a concentration to prevent precipitation of the nucleic acid in the presence of the cationic detergent, and
iii) at least one water-insoluble non-ionic polymeric adsorbent;

b) incubating the extraction mixture and forming a precipitate which comprises the cationic detergent and inhibitors contained in the sample and separating the formed precipitate from the supernatant, thereby providing an inhibitor depleted supernatant which comprises the extracted nucleic acid.

Also provided is a kit for performing said method.

## Description

### FIELD OF THE INVENTION

The present invention pertains to a method for extracting a target nucleic acid such as DNA from a sample, in particular from an inhibitor rich sample such as a food sample. Furthermore, a kit is provided which can be used to extract a target nucleic acid from such samples.

### BACKGROUND OF THE INVENTION

Many methods for extracting and isolating a target nucleic acid such as e.g. DNA are known in the prior art. The obtained target nucleic acid is usually subsequently analysed by an analytical method. A prerequisite for most analytical methods is that the target nucleic acids are made available in such a way that they are provided in an amount capable of being analysed and with sufficient purity for the intended analytical method. Many analytical methods are, however, very sensitive to inhibitors contained in the isolated target nucleic acid. Examples of such analytical methods are amplification based methods, such as e.g. PCR. If inhibitory components are still present in the isolated target nucleic acid, this reduces the sensitivity and accuracy of the analytical method.

Effectively reducing the amount of inhibitory components (also referred to herein as "inhibitors") during preparation of the target nucleic acid is a major challenge when processing inhibitor rich samples, such as e.g. food samples or plant derived samples.

Food samples, as one example, represent a complex and challenging target for food diagnostics and routine safety testing. E.g. industrially processed foods are regularly analysed as quality control. Processed foods are particularly challenging to process and analyze based on their nucleic acid content, because in contrast to raw materials, they exhibit a multitude of biological as well as chemical inhibitors and additives. Efficient extraction of nucleic acids such as in particular DNA contained in food from strongly processed food types, however, is a crucial prerequisite for the determination of e.g. GMOs (genetically modified organisms), allergen contents or authentication of plant and animal species. The major hurdle in nucleic acid extraction from processed foods originates from a dense matrix of highly inhibitory biological and chemical compounds. Next to proteins, polysaccharides, polyphenols and fats, a wide range of chemical flavor enhancers, antioxidants, salts or coloring agents complicate the overall inhibitor spectrum. Carry-over of such inhibitors into the subsequent analytical method such as a PCR reaction leads to strong inhibition of the analytical method. This can distort the analytical result. Therefore, as these inhibitors strongly influence the stability and reliability of the subsequent analytical method such as PCR, the extraction protocols for processed foods have to be specifically adjusted to deplete inhibitors and render isolated nucleic acids suitable for analysis.

Therefore, efficient inhibitor removal signifies a major challenge in the investigation of nucleic acids from inhibitor rich samples, such as in particular processed food types.

A method that is widely used in the art for extracting nucleic acids from inhibitor rich samples is based on the use of a cationic detergent, such as CTAB. Depending on the salt concentration in the extraction mixture, CTAB may complex with nucleic acids (under low-salt conditions) or may complex with the inhibitors, such as polysaccharides, proteins or plant-metabolites (under high-salt conditions). For extracting nucleic acids from inhibitor rich samples, high-salt conditions are regularly used. The cationic detergent forms a precipitate with the inhibitors contained in the sample, leaving the extracted nucleic acids in the supernatant from which the nucleic acids can then subsequently be isolated. CTAB based extraction methods are widely used and e.g. are also recommended as the method of choice by many official authorities (see e.g. the official German method, proposed by the CEN (Central European Committee for Standardization) in 2002; § 64 LFGB L 00.00-31).

A commercially available CTAB based extraction method is the DNeasy® *mericon*™ Food Kit (QIAGEN) which is suitable for isolating total nucleic acids from a broad range of food sample types. Here, CTAB is used in combination with Proteinase K to first digest also compact food samples and to subsequently precipitate proteins with simultaneous precipitation of other cellular and food-derived inhibitors. Inhibitors are precipitated, what is assisted by centrifugation, while the extracted DNA remains in solution. Subsequently, a chloroform extraction is performed to remove remaining CTAB-protein, CTAB-debris, or CTAB-polysaccharide complexes along with other lipophilic inhibitors into the organic phase. The obtained aqueous phase containing the DNA is processed further in order to isolate the DNA. For this purpose an isolation method is used that involves a silica solid phase and a chaotropic binding buffer. The obtained DNA is ready for use in a subsequent PCR reaction.

Furthermore, CTAB based extraction methods are described in the prior art that aim at improving the inhibitor removal capacity. E.g. polyvinylpyrrolidone (PVP) is used in addition to the cationic detergent CTAB in order to improve the inhibitor removal as is e.g. described in Khanuja et al., 1999 (Plant Molecular Biology Reporter 17: 1-7, 1999) and Brown et al, 1998 (Cucurbit Genetics Cooperative Report 21: 46-47).

However, the inhibitor depletion capacity of the CTAB based extraction buffers known from the prior art and thus the processable amount of sample per preparation is limited. This is particularly problematic when extracting nucleic acids such as DNA from foods with low nucleic acid content but high inhibitor content (e.g. ketchup) because the inhibitor removal capacity of the extraction buffer can be exceeded during the food preparation. In such cases, inhibitors are not sufficiently removed during the preparation and the isolated nucleic acids are not sufficiently pure for the subsequent analysis. Furthermore, considering the low amount of contained nucleic acids, a larger amount of sample would need to be processed in order to obtain a sufficient amount of nucleic acids for a subsequent analysis. A larger amount of sample in turn, however, means that the inhibitor amount is also increased. Thus, there is a demand to increase the amount of sample that can be processed per volume of extraction buffer and hence preparation, in particular when intending to process inhibitor rich samples such as food samples, in order to achieve higher amounts of nucleic acids for enhanced sensitivity and reliability of a subsequently performed analytical method such as in particular PCR based methods. This is of particular interest e.g. in allergen or GMO detection, in which traces of DNA have to be detected in an excess matrix of strongly inhibitory compounds. An improved inhibitor removal technology would enable a general upscale of processed food per preparation to achieve higher nucleic acid (in particular DNA) yields, while ensuring sensitive and inhibitor unbiased PCR detection methods.

It is one object of the present invention to provide a cationic detergent based nucleic acid extraction method that improves the removal of inhibitors that are contained in the sample. In particular, it is an object to provide an improved method for extracting DNA from a food sample.

### SUMMARY OF THE INVENTION

The present invention is based on the finding that the inhibitor removal capacity of a cationic detergent based nucleic acid isolation method can be significantly improved, if at least one water-insoluble non-ionic polymeric adsorbent is used in combination with the cationic detergent which forms a precipitate with the inhibitors contained in the sample. As is shown by the examples, the nucleic acid extraction method according to the invention provides improved results compared to prior art methods.

According to a first aspect of the present invention, a method for extracting a target nucleic acid from a sample and depleting inhibitors contained in the sample is provided, comprising the following steps:
a) preparing an extraction mixture by contacting the sample with
   i) at least one cationic detergent capable of forming a precipitate with inhibitors contained in the sample,
   ii) at least one salt, wherein the salt is contained in the extraction mixture in a concentration to prevent precipitation of the target nucleic acid in the presence of the cationic detergent, and
   iii) at least one water-insoluble non-ionic polymeric adsorbent;
b) incubating the extraction mixture and forming a precipitate which comprises the cationic detergent and inhibitors contained in the samples and separating the formed precipitate and the polymeric adsorbent from the supernatant, thereby providing an inhibitor depleted supernatant which comprises the extracted target nucleic acid.

According to a second aspect of the present invention a kit for extracting a target nucleic acid from a sample is provided, which comprises
a) an extraction composition comprising
   i) at least one salt in a concentration selected from 0.7 M to 3 M, 0.8M to 2.75M, 0.9M to 2.5M, 1 M to 2 M, 1.1 M to 1.75M and 1.2 M to 1.5 M; and
   ii) at least one cationic detergent in a concentration selected from 1% to 5%, 1.25% to 4%, 1.5% to 3.5%, 1.7% to 3%, 1.8% to 2.75%, 1.9% to 2.5% and 2.0% to 2.2%, wherein the cationic detergent is capable of forming a precipitate with inhibitors contained in the sample;
b) at least one water-insoluble non-ionic polymeric adsorbent; and
c) optionally a proteolytic enzyme.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

### BRIEF DESCRIPTION OF THE FIGURES

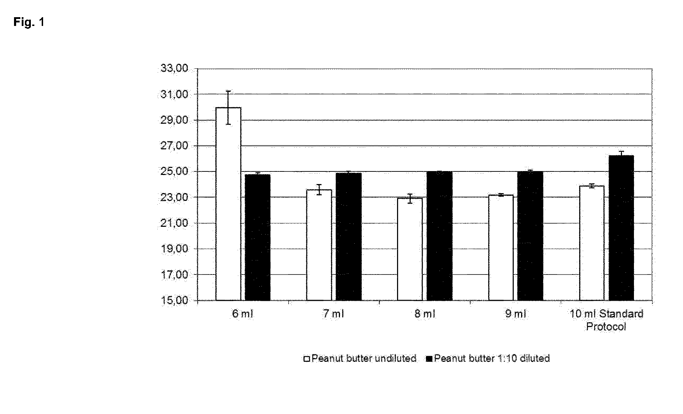

**Figure 1****:** Processing of peanut butter according to the DNeasy *mericon* Food Standard Protocol. As reference, 10ml food lysis buffer was used applying original reaction volumes. Within the protocol, the CTAB buffer volume that was used per preparation was titrated down from initially 10 ml to 6 ml. The supernatant volume subsequently transferred for chloroform extraction was additionally increased from 700 µl to 1 ml. Undiluted as well as 1:10 diluted eluates were measured.

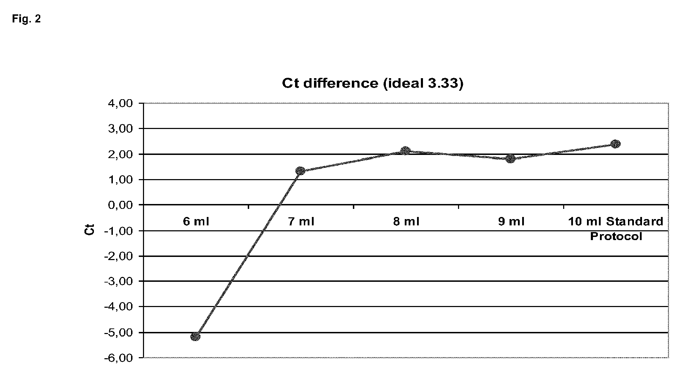

**Figure 2****:** Differences between the Ct values of the undiluted (set at 0) and 1:10 diluted DNA extraction eluate. An expected Ct value of 3.33 indicates an inhibitor free PCR reaction.
Differences below 3.33 indicate inhibitor carry-over from the sample preparation. When the 1:10 dilution exhibits a lower Ct value than the undiluted sample, this indicates that the PCR reaction is strongly inhibitor loaded.

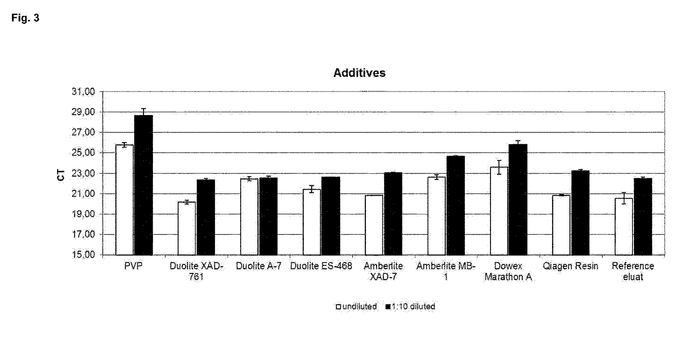

**Figure 3****:** PCR results of a series of peanut butter DNA extraction experiments (7 ml CTAB buffer). Several inhibitor removal additives, including non-ionic polymeric adsorbents, ion exchanger materials and PVP, were added to the initial food lysis solution with the aim to extend the limited inhibitor removal capacities of the CTAB buffer. Undiluted as well as 1:10 diluted preparation eluates were measured, using a generic plant assay.

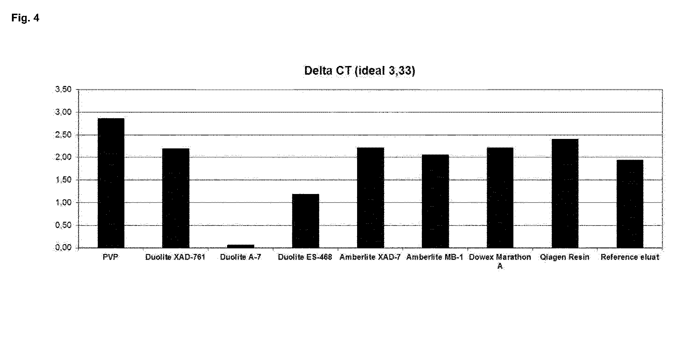

**Figure 4****:** Detailed illustration of the Ct difference between the undiluted (set at 0) and 1:10 diluted preparation eluates from figure 3. An expected Ct value approximating 3.33 indicates an inhibitor free PCR reaction. Differences below 3.33 indicate inhibitor carry-over from the sample preparation.

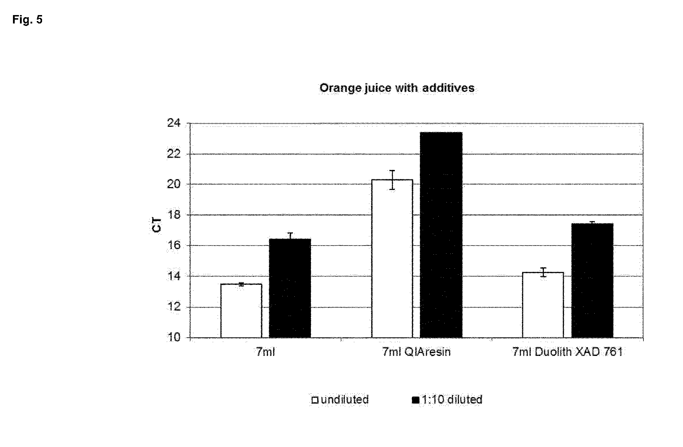

**Figure 5****:** Application of several surface functionalized materials in combination with a lowinhibitor sample matrix (orange juice). A DNA-binding effect would lead to higher Ct values and no DNA-binding effect was observed for the polymeric adsorbent Duolite XAD-761.

### DETAILED DESCRIPTION OF THE INVENTION

### METHOD

According to a first aspect of the present invention, a method for extracting a target nucleic acid from a sample and depleting inhibitors contained in the sample is provided, comprising the following steps:
a) preparing an extraction mixture by contacting the sample with
   i) at least one cationic detergent capable of forming a precipitate with inhibitors contained in the sample,
   ii) at least one salt, wherein the salt is contained in the extraction mixture in a concentration to prevent precipitation of the target nucleic acid in the presence of the cationic detergent, and
   iii) at least one water-insoluble non-ionic polymeric adsorbent;
b) incubating the extraction mixture and forming a precipitate which comprises the cationic detergent and inhibitors contained in the sample and separating the formed precipitate and the polymeric adsorbent from the supernatant, thereby providing an inhibitor depleted supernatant which comprises the extracted target nucleic acid.

The individual steps of said method and preferred embodiments thereof are subsequently described in detail.

### Step a)

In step a) an extraction mixture is prepared by contacting the sample with at least one cationic detergent which is capable of forming a precipitate with inhibitors contained in the sample, at least one salt and at least one water-insoluble polymeric adsorbent. The components can be added in any order or also at the same time. As will be described in further detail below, the sample is preferably a disrupted and/or homogenized sample. The sample can in particular be an inhibitor rich sample, such as e.g. a food sample.

### The cationic detergent

The extraction method of the present invention uses at least one cationic detergent in order to complex inhibitors contained in the sample in the presence of a salt. Typical inhibitors include polysaccharides, proteins, fats, polyphenols, antioxidants, chemical flavor enhancers and/or plant metabolites. Which type or types of inhibitors are present depends on the nature of the processed sample. The cationic detergent forms a precipitate with these inhibitors, thereby allowing their depletion.

The cationic detergent is according to one embodiment a quarternary ammonium salt. A cationic detergent can be used which comprises a permanently charged quaternary ammonium cation as polar head group. Preferably, the cationic detergent is an alkyltrimethylammonium salt. It may comprise a halogenide as counter ion, such as bromide or chloride. As unpolar part, the cationic detergent preferably comprises at least one alkyl group having a chain length of from 6 to 22 carbon atoms, 8 to 20 carbon atoms, 10 to 18 carbon atoms, 12 to 18 carbon atoms or 16 carbon atoms. According to one embodiment, the cationic detergent only comprises one respective alkyl chain. Preferably, the cationic detergent is selected from the group consisting of cetyl trimethyl ammonium bromide (CTAB), tetra decyl trimethyl ammonium bromide (TTAB) and dodecyl trimethyl ammonium bromide (DTAB) or the corresponding compounds comprising a chloride instead of bromide. Also a combination of two or more of the respective cationic detergents can be used. CTAB is most preferably used as cationic detergent because CTAB is also widely used in the prior art for depleting inhibitors from different types of samples and is also accepted as suitable cationic detergent by many official authorities.

The cationic detergent is used in a concentration wherein it can complex inhibitors contained in the sample. Suitable concentrations and concentration ranges for the cationic detergent in the extraction mixture are known to the skilled person from prior art methods and examples are also described below in conjunction with the extraction composition, which can be used to establish in the extraction mixture the conditions required for precipitating with the cationic detergent inhibitors contained in the sample and extracting the target nucleic acids. It is referred to the respective disclosure.

### The salt

The extraction mixture furthermore comprises at least one salt. As discussed above in the background, the salt concentration in the extraction mixture is important in order to achieve that the cationic detergent complexes and hence precipitates inhibitors contained in the sample, but substantially does not precipitate the target nucleic acid. Therefore, the salt is contained in the extraction mixture in a concentration that is sufficiently high to prevent precipitation of the target nucleic acid. This ensures that the target nucleic acid substantially remains in the supernatant and is not precipitated together with the inhibitors. This is achieved by using the salt that is added for extraction in a fairly high concentration, preferably at least 0.5M, more preferred at least 0.7M, as is well-known in the prior art. Suitable salt concentrations are also described below in conjunction with the extraction composition that is used in order to prepare the extraction mixture. The salt can be a chaotropic salt or a non-chaotropic salt. Also combinations of salts can be used.

According to one embodiment, the salt is a non-chaotropic salt. Non-chaotropic salts include, but are not limited to, alkali metal salts, alkaline earth metal salts and ammonium salts. According to one embodiment, the at least one salt is an alkali metal salt such as for example a lithium, a sodium or a potassium salt. The alkali metal salt can be an alkali metal halogenide. The use of alkali metal chlorides, such as sodium chloride or potassium chloride, is preferred. Sodium chloride is also widely used in prior art methods in order to establish the salt concentration in the extraction mixture. Therefore, sodium chloride is a preferred salt.

The salt is contained in the extraction mixture in a concentration sufficiently high to substantially prevent precipitation of the target nucleic acid in the presence of the cationic detergent. Suitable concentration ranges are known to the skilled person and are also described below in conjunction with the extraction composition, which can be used in order to prepare the extraction mixture. It is referred to the respective disclosure.

### The water-insoluble non-ionic polymeric adsorbent

The extraction mixture furthermore comprises at least one water-insoluble non-ionic polymeric adsorbent. It was found and is shown by the examples that including a respective polymeric adsorbent in the extraction mixture significantly improves the precipitation and hence removal of inhibitors. As is demonstrated by the examples, higher sample amounts can be processed with the same amount of extraction buffer volume and/or the amount of extraction buffer volume can be reduced because the inhibitor removal capacity is increased when including a respective polymeric adsorbent in the extraction composition. The water-insoluble polymeric non-ionic adsorbent therefore significantly improves the depletion of inhibitors. Without wishing to be bound in theory, it is believed that the polymeric adsorbent functions as a precipitation seed, helping efficient precipitation of formed but still dissolved cationic detergent/inhibitor complexes, thereby supporting and improving the formation of precipitates comprising the cationic detergent and the inhibitors. Furthermore, a hydrophobic interaction between e.g. the long, hydrophobic alkyl groups of the cationic detergent and the non-ionic polymeric adsorber material could occur, in particular with non-ionic functional groups of the non-ionic polymeric adsorber material. The cationic detergent, such as for example CTAB, binds the inhibitors, and the respectively formed complexes could be bound via the alkyl groups of the cationic detergent to the non-ionic polymeric adsorbent. Irrespective of the underlying mechanism, the examples demonstrate that including such non-ionic polymeric adsorbent in the extraction mixture results in that the precipitates are formed more effectively and/or can be separated more efficiently from the supernatant containing the target nucleic acid. In any case, combining a cationic detergent based precipitation with the use of a water-insoluble non-ionic polymeric adsorbent significantly improves the removal of inhibitors and thereby makes the method according to the present invention superior to prior art methods. In particular, higher amounts of sample material can be processed per preparation, what is an important advantage in particular if the nucleic acid content in the sample is low as it is the case with many food samples, in particular processed food samples.

Exemplary and preferred characteristics of the non-ionic polymeric adsorbent are described in the following:

The non-ionic polymeric adsorbent can be a non-polar or polar adsorbent. It can be a resin. If a polar non-ionic polymeric adsorbent is used, weakly polar adsorbents are preferred, suitable examples are described below. According to one embodiment, the polymeric adsorbent has a hydrophilic surface.

The non-ionic polymeric adsorbent may comprise one or more surface functionalities selected from acryl, acrylic ester, phenol, phenolic hydroxyl and/or methylol groups and hydroxyl-methyl functionalities. E.g. acryl, phenolic hydroxyl and methylol surface groups can account for the hydrophilic properties.

The non-ionic polymeric adsorbent may exhibit in a polar solvent such as water a non-polar or hydrophobic behavior. The hydrophobicity is most pronounced with adsorbents having a low dipole moment. In non-polar solvents, the polymeric adsorbent may exhibit polar or hydrophilic properties. These polarities are pronounced with polymeric adsorbents having a higher dipole moment, as is the case e.g. with acrylic adsorbents (such as e.g. aliphatic acrylic ester adsorbents) and phenolic adsorbents. The dipole moment of the non-ionic polymeric adsorbent used can lie within the range of 0.5 to 2, such as 0.75 to 2, 1 to 2, 1.25 to 2 and 1.5 to 2. Particularly preferred is a dipole moment in the range of 1.5 or 1.6 to 1.9. As is demonstrated by the examples, the non-ionic polymeric adsorbents XAD-761 and XAD-7 have particularly good inhibitor depletion characteristics. The dipole moment of these adsorbents lies within the described ranges. E.g. XAD-7 is a moderately polar resin having a dipole moment in the range of approx. 1.7-1.9, in particular 1.8. XAD-7 comprises a crosslinked polyacrylate matrix with no additional functional groups.

The polymeric adsorbent can be a cross-linked polymer having a matrix that is based e.g. on polystyrene, phenol-formaldehyde, or acrylate. Excerpts of exemplary matrices are shown below:

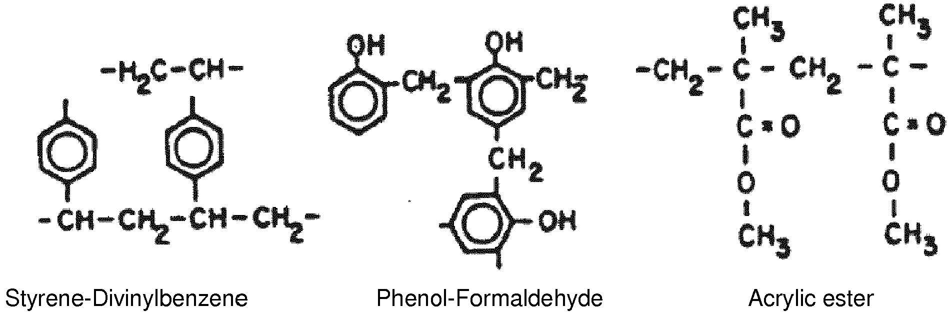

Exemplary crosslinking agents may include divinylbenzene, polyfunctional aliphatic monomers such as methacrylic acid, acrylic acid or derivatives thereof. According to one embodiment, the polymeric adsorbent is a self-crosslinked polymer. It can be e.g. a self-crosslinked acrylic ester homopolymer from monomers having polyfunctional groups, such as at least three methacrylate groups, capable of self-crosslinking, e.g., trimethylolpropane trimethacrylate and pentaerythritol tetramethacrylate, etc. The non-ionic polymeric adsorbent can be aliphatic. According to one embodiment, a cross-linked acrylic ester polymer is used wherein the functional groups have a dipole moment in the range of about 1.6-2.0, preferably 1.7 to 1.9, more preferably 1.8. A suitable example is XAD-7.

The water-insoluble non-ionic polymeric adsorbent is preferably porous. A porous structure is advantageous, because it provides an internal surface area which provides a higher interaction surface. This may improve the removal of inhibitors inhibitor what is an important advantage when processing inhibitor rich samples. According to one embodiment, the non-ionic polymeric adsorbent is a porous, spherical resin. The average pore diameter may lie in a range selected from 50 to 1,000 Angstrom, e.g. 60 to 750 Angstrom or 75 to 700 Angstrom. According to one embodiment, the average pore diameter lies in the range of 350 to 1,000 Angstrom, for example 400 to 800 Angstrom or 500 to 700 Angstrom. An example of a respective non-ionic polymeric adsorbent is XAD-761. According to another embodiment, said non-ionic polymeric adsorbent has an average pore diameter that lies in the range of 50 to 300 Angstrom, e.g. within the range of 60 to 250 Angstrom, 70 to 200 Angstrom or 75 to 150 Angstrom. An example of a respective non-ionic polymeric adsorbent is XAD-7.

According to one embodiment, the non-ionic polymeric adsorbent has a BET surface area (m²/g) that lies in a range selected from 100 to 1000 m²/g and 125 to 750 m²/g. According to one embodiment, the BET surface are lies in the range of 100 to 500 m²/g, preferably 125 to 350 m²/g. An example of a respective non-ionic polymeric adsorbent is XAD-761. According to another embodiment, said non-ionic polymeric adsorbent has a surface area of 200 to 700 m²/g, 250 to 650 m²/g or 300 to 550 m²/g. An example of a respective polymeric adsorbent is XAD-7.

According to one embodiment, the non-ionic polymeric adsorbent is a porous, poly-phenolic adsorbent. The matrix of the adsorbent may be provided by a phenol-formaldehyde polymer which preferably is cross-linked. A suitable example that can be used according to the invention is XAD-761, which is a cross-linked phenol-formaldehyde polymer. According to one embodiment, the non-ionic polymeric adsorbent is a porous, aliphatic, acrylic ester polymer. A suitable polymeric adsorbent is for example the resin XAD-7, see also CAS No.: 37380-43-1. A similar suitable product is Diaion HP2MG.

The non-ionic polymeric adsorbent used herein may be provided in the form of particles or granules, such as spherical particles. The particles may have a mean diameter selected from the ranges 100 to 2000µm, 150 to 1750µm, 200 to 1500µm, 300 to 1250µm and 400 to 1000µm. The particles can be magnetic or non-magnetic. When using magnetic particles, separation of the precipitate and the particular polymeric adsorbent can be assisted by a magnetic field. However, non-magnetic particles may also be used because the precipitate that is formed by the cationic detergent and the inhibitors is sedimented during the process to ensure efficient precipitate formation and separation. During this process, the water-insoluble polymeric adsorbent is likewise sedimented which subsequently facilitates the separation of the formed precipitate and the polymeric adsorbent from the remaining sample.

According to one embodiment, the non-ionic polymeric adsorbent is a porous, cross-linked phenol-formaldehyde polymer having an average pore diameter in the range of 350 to 1,000 Angstrom, 400 to 800 Angstrom or 500 to 700 Angstrom and having a BET surface in the range of 100 to 500 m²/g or 125 to 350 m²/g. It preferably is provided in the form of particles. These particles may have a mean diameter that lies in the range of 200 to 1500µm, preferably 300 to 1250µm and more preferably 400 to 1000µm. A suitable example of such a polymeric adsorbent is XAD-761. Alternatively or additionally, a porous, aliphatic, acrylic ester polymer may be used as non-ionic polymeric adsorbent which has an average pore diameter in the range of 50 to 300 Angstrom, 70 to 200 Angstrom or 75 to 150 Angstrom, a BET surface in the range of 200 to 700 m²/g, 250 to 650 m²/g or 300 to 550 m²/g and a dipole moment in the range of 1.5 to 2.0 or 1.6 to 1.9. It preferably is provided in the form of particles. These particles may have a mean diameter that lies in the range of 200 to 1500µm, preferably 300 to 1250µm and more preferably 400 to 1000µm. A suitable example of such a polymeric adsorbent is XAD-7.

The water-insoluble non-ionic polymeric adsorbent used in the present method has under the used extractions conditions substantially no nucleic acid binding capacity. This avoids a loss of target nucleic acids during the precipitation step. Thus, the polymeric adsorbent should have no or a rather low basic or acidic ion exchange capacity. According to one embodiment, it does not comprise basic or acidic ion exchange functionalities capable of binding nucleic acids under the used extraction conditions. Preferably, the used non-ionic polymeric adsorbent lacks any ionic functional groups.

The amount of non-ionic polymeric adsorbent in the extraction mixture that is suitable to support the depletion of inhibitors can be determined by the skilled person based on routine experiments. The amount of polymeric adsorbent should be high enough to achieve an additional depletion of inhibitors while binding of target nucleic acids should be substantially avoided. According to one embodiment, the amount of non-ionic polymeric adsorbent (in mg) that is added per ml extraction composition (that is used to prepare the extraction mixture) is selected from 50 to 150 mg per ml extraction composition, 55 to 140 mg per ml extraction composition, 60 to 125 mg per ml extraction composition, 65 to 115 mg per ml extraction composition and 70 to 100 mg per ml extraction composition. E.g. the sample can be contacted with 5 to 7ml extraction composition and 500 to 650 mg non-ionic polymeric adsorbent in order to prepare the extraction mixture.

### Further embodiments

According to one embodiment, the sample, which is preferably a disrupted and/or homogenized sample, is contacted with an extraction composition which comprises the at least one salt and the at least one cationic detergent. E.g. an extraction buffer can be used as extraction composition as it is also common in the prior art.

According to one embodiment, the extraction composition (and/or the extraction mixture) comprises the salt in a concentration selected from 0.7M to 3M, 0.8M to 2.75M, 0.9M to 2.5M, 1 M to 2 M, 1.1 M to 1.75M and 1.2 M to 1.5 M. Such salt concentrations are regularly used in the extraction composition of standard CTAB based extraction protocols. As described above, alkali metal salts such as alkali metal halogenides can be used as salts. Sodium chloride is particularly suitable and commonly used.

According to one embodiment, the extraction composition (and/or the extraction mixture) comprises the cationic detergent in a concentration selected from 1 to 5 %, 1.25 to 4%, 1.5% to 3.5%, 1.7% to 3%, 1.8% to 2.75%, 1.9% to 2.5% and 2.0 to 2.2 %. Such cationic detergent concentrations are regularly used in extraction compositions of CTAB based extraction protocols in order to prepare the sample for extraction and provide a sufficient amount of the cationic detergent in the extraction mixture in order to effectively precipitate inhibitors contained in the sample. Suitable examples for cationic detergents were described above, CTAB being particularly preferred.

The extraction composition may have a pH selected from 7 to 9, 7.25 to 7.25 to 8.75, 7.5 to 8.5, 7.75 to 8.25 and 7.9 to 8.1. According to one embodiment, the pH of the extraction mixture is in the same range.

The extraction composition is preferably an extraction buffer. Therefore, the extraction composition may comprise at least one buffer substance. The buffer substance should be capable of buffering the extraction composition at the desired pH. The pH buffer substance can be comprised in the extraction composition in a concentration of e.g. 10 mM to 250 mM, preferably 50 mM to 150 mM. By way of example, the buffer substance can be selected from the group consisting of tris(hydroxymethyl)aminomethane (TRIS), N-(tri(hydroxymethyl)methyl)glycine (Tricine), N,N-bis(2-hydroxyethyl)glycine (BICINE), N-(2-hydroxyethyl)piperazine-N'-(2-ethanesulphonic acid) (HEPES), piperazine-1,4-bis(2-ethanesulphonic acid) (PIPES), N-cyclohexyl-2-aminoethanesulphonic acid (CHES), 2-(N-morpholino)ethanesulphonic acid (MES), 3-(N-morpholino)propanesulphonic acid (MOPS) and phosphate buffer.

According to one embodiment, the extraction composition furthermore comprises at least one chelating agent. Suitable chelating agents include but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA). According to a preferred embodiment, EDTA is used. The term "EDTA" indicates inter alia the EDTA portion of an EDTA compound such as, for example, K2EDTA, K3EDTA or Na2EDTA. Using a chelating agent such as EDTA has the advantageous effect that nucleases such as DNases and RNases are inhibited. The extraction composition may comprise the chelating agent in a concentration selected from 10 mM to 50 mM, 15 mM to 35 mM, 17 mM to 30 mM, 18mM to 25 mM and 19 mM to 23 mM.

According to one embodiment, the extraction composition is an aqueous composition comprising
i) a cationic detergent, preferably CTAB, in a concentration from 1.7% to 3%, preferably 1.8% to 2.75%, more preferred 1.9% to 2.25%
ii) a salt, preferably a sodium salt, in a concentration selected from 1 M to 2 M, preferably 1.1 M to 1.75M, more preferred 1.2 M to 1.5 M;
iii) a buffering agent; and
iv) optionally a chelating agent, preferably EDTA, e.g. in a concentration selected from 10 mM to 35 mM, preferably 15 mM to 30 mM, more preferably 18 mM to 25 mM.

Suitable extraction buffers described in the prior art e.g. contain a cationic detergent, such as CTAB, in a concentration from 15 to 25 g/l (1.5-2.5%), a salt, such as sodium or potassium chloride in a concentration of 1 M to 2 M, a buffering agent (such as TRIS) preferably in a concentration of 0.05 to 0.2 M and a chelating agent such as EDTA in a concentration of 0.01 to 0.03 M. The pH-value can be between 7 to 9, preferably 7.5 to 8.5, more preferably around 8. Also suitable is an extraction buffer comprising CTAB (20 g/l, sodium chloride (1.4 M), TRIS (0.1 M), Na2EDTA (0.02 M) or a variant thereof, wherein the concentration of the contained agents varies by 1 % - 30 %, 1% - 20% or 1% to 15%.

The extraction composition is contacted with the sample to prepare the extraction mixture. Suitable ratios of extraction composition/sample are known from the prior art and can also be determined by the skilled person. An advantage of the present invention is that the amount of sample can be increased per volume of extraction buffer. E.g. according to the prior art standard, 2g sample are processed with 10ml extraction composition. The method of the invention can due to the improved inhibitor depletion performance process the same amount of sample (2g) with just 7ml extraction buffer or even 5ml extraction buffer, depending on the processed sample type. This allows e.g. to significantly reduce the amount of extraction buffer that is used for a sample of 2g or to significantly increase the amount of sample that is processed with the standard 10ml extraction composition. According to one embodiment, the amount of sample per ml extraction composition is selected from 0.1g to 0.5 g sample per ml extraction composition, 0.15g to 0.45g sample per ml extraction composition, 0.2g to 0.4g sample per ml extraction composition, 0.225g to 0.375g sample per ml extraction composition, 0.235g to 0.35g sample per ml extraction composition, 0.245g to 0.325g sample per ml extraction composition and 0.25g to 0.3g sample per ml extraction composition. E.g. when contacting the extraction composition with the sample, 5 ml extraction composition can be used per 1 g of sample. However, advantageously, less than 5 ml extraction composition can be used per 1 g of sample, such as 4.5 ml or less, 4 ml or less, 3.5 ml or less, 3 ml or less or even 2.5 ml of the extraction composition per 1 g of the sample, depending on the sample type. The extraction composition may be used in an amount of from 2.5 to 5 ml per 1 g of the sample, such as 2.75 to 4.75 or 3 to 4.5 ml extraction composition per 1 g sample or from 3.25 to 4.25 or 3.5 to 4 ml extraction composition per 1 g sample. According to one embodiment, the extraction composition is contacted with the sample in a ratio of from 10 : 4 to 10 : 1 (volume of the extraction composition : weight of the sample (e.g. ml : g or µl : µg), such as from 10 : 3.5 to 10 : 2, 10 : 3.25 to 10 : 2.25, 10 : 3 to 10 : 2.5 or 6 : 2 to 9 : 2 or 7 : 2 to 8 : 2.

The salt and the cationic detergent comprised in the extraction composition may be comprised in the same concentration in the extraction mixture as is described herein for the extraction composition (see e.g. above). Whether the addition of the sample leads to a dilution of the extraction composition depends on the type of sample. E.g. a solid sample usually has a rather low or even no dilution effect. In such case, the concentration of the salt and the cationic detergent present in the extraction composition is essentially not or at least not significantly reduced in the mixture. Even liquid samples usually have a moderate dilution effect assuming e.g. for calculation purposes that 1ml liquid sample corresponds to 1g sample. According to one embodiment, the concentration of the salt and the cationic detergent present in the extraction composition is reduced in the extraction mixture that is obtained after contacting the extraction composition with the sample by a factor of up to 1.4, in particular by a factor selected from 1.05 to 1.4, 1.1 to 1.35, 1.15 to 1.3 and 1.2 to 1.25. In one embodiment, the salt of the extraction composition is present in the extraction mixture in a concentration selected from 0.5 M to 3 M, 0.55 M to 2.75 M, 0.6 M to 2.5 M, 0.7 M to 2 M, 0.8 M to 1.75 M and 1.0 M to 1.5 M. The cationic detergent of the extraction composition may be present in the extraction mixture in a concentration selected from 0.7 to 5%, 0.85 to 4%, 1% to 3.5%, 1.2% to 3%, 1.4% to 2.5%, 1.5% to 2.25% and 1.7 to 2%.

As described herein, the sample is preferably a disrupted and/or homogenized sample. The sample is usually lysed prior to and/or when the extraction mixture is prepared. As is shown by the examples, lysis of the sample, which can be a homogenized sample, can also occur in, respectively can be assisted by the extraction composition. The term "lysis" as used herein refers to the disruption, degradation and/or digestion of a sample. In a respective lysis step, nucleic acids can be released from cells and/or can be freed from other sample components such as e.g. proteins. Herein, it is referred to a respective step to disrupt, degrade and/or digest a sample generally as "lysis step", irrespective of whether nucleic acids are released from cells or whether the lysis is performed in order to release nucleic acids e.g. from proteins or other substances comprised in the sample or in order to degrade the sample. Several methods are known that allow to achieve an efficient lysis of different sample materials. Suitable lysis methods include but are not limited to mechanical, chemical, physical or enzymatic actions on the sample. Examples of respective lysis steps include but are not limited to grinding the sample in a bead mill, sonication, surface acoustic waves (SAW), repeated cycles of freezing and thawing, heating, the addition of detergents and/or the addition of protein degrading compounds such as e.g. protein degrading enzymes. Lysis of the sample in the extraction composition can be promoted and hence assisted by additional means, e.g. by performing a heating step. Any lysis procedure used is compatible with the cationic detergent based extraction.

According to one embodiment, the extraction mixture comprises a protein degrading compound in order to support the lysis of the sample. According to a preferred embodiment, the protein-degrading compound is a proteolytic enzyme. A proteolytic enzyme refers to an enzyme that catalyzes the cleavage of peptide bounds, for example in proteins, polypeptides, oligopeptides and peptides. Exemplary proteolytic enzymes include but are not limited to proteinases and proteases in particular subtilisins, subtilases, alkaline serine proteases and the like. Subtilases are a family of serine proteases, i.e. enzymes with a serine residue in the active side. Subtilisins are bacterial serine protease that has broad substrate specificities. Exemplary subtilisins include but are not limited to proteinase K, proteinase R, proteinase T, subtilisin, subtilisin A, QIAGEN Protease and the like. Discussions of subtilases, subtilisins, proteinase K and other proteases may be found, among other places in Genov et al., Int. J. Peptide Protein Res. 45: 391 -400, 1995. Preferably, the proteolytic enzyme is proteinase K. The proteolytic enzyme may be added separately from the extraction composition to the sample or may be contained in the extraction composition. Proteinase K is commonly used in CTAB based extraction protocols in order to support the lysis of the sample. Furthermore, one or more cell wall digesting enzymes can be used to support the lysis such as e.g. lysozyme, zymolase and/or pektinases. These parameters can also vary from sample to sample and can be adjusted by the skilled person.

### Step b)

Step b) comprises incubating the obtained extraction mixture and forming a precipitate, which comprises the cationic detergent and inhibitors contained in the sample and separating the formed precipitate and the non-ionic polymeric adsorbent from the supernatant which contains the target nucleic acid. As is described herein, the precipitation conditions are such that only little target nucleic acids get lost, if at all.

According to one embodiment, the extraction mixture is agitated in step b) during incubation in order to support the formation of the precipitates. Accordingly, precipitation can be assisted by agitation. Agitation includes, but is not limited to vortexing, shaking, inverting and pipetting up and down. Furthermore, the extraction mixture may be cooled, for example stored on ice, after incubation in order to enhance inhibitor precipitation. According to one embodiment, the extraction mixture is cooled down to room temperature or lower.

According to one embodiment, the extraction mixture is incubated at an elevated temperature, preferably at a temperature that lies in a range of 45°C to 70°C, preferably 50°C to 65°C. This supports the lysis of the sample. Incubating the extraction mixture at such elevated temperature, in particular in combination with agitation, is particularly advantageous in case a proteolytic enzyme is included in the extraction mixture as these measures support the activity of the proteolytic enzyme.

According to one embodiment, the extraction mixture which comprises a proteolytic enzyme is incubated for approximately 15 to 60 minutes, preferably 20 to 45 minutes in order to digest the sample. According to one embodiment, the extraction mixture is incubated at an elevated temperature, preferably is also agitated, and afterwards, the sample is cooled to support the formation of the precipitates. E.g. the extraction mixture may be cooled down at least to room temperature.

As is described herein, the precipitate that is formed comprises the cationic detergent and inhibitors contained in the sample. If not sufficiently removed, such inhibitors can disturb the subsequent detection of the extracted and isolated target nucleic acid. If such inhibitors are contained in the isolated nucleic acid fraction, they can disturb downstream analysis or detection methods. As is described herein, depletion of the inhibitors is surprisingly more effective when the extraction mixture additionally comprises a polymeric water-insoluble non-ionic adsorbent as taught herein. Without wishing to be bound in theory, the polymeric adsorbent may function as precipitation seed, thereby supporting the formation of the precipitates. Furthermore, it might provide an interaction surface for the formed complexes, thereby also supporting precipitate formation and/or subsequent separation of the formed precipitates.

Precipitate formation and separation of the precipitates can be assisted by various means such as e.g. sedimentation and/or centrifugation. Furthermore, the formed precipitate and the polymeric adsorbent may also be separated by filtration to provide the supernatant which comprises the target nucleic acid. The term "supernatant" is therefore not limited to a certain mode of action to separate the precipitate. Thus, the term "supernatant" as used herein e.g. encompasses embodiments wherein the precipitate is collected at the bottom of a vessel and wherein the remaining sample is removed as supernatant as well as embodiments wherein the extraction mixture is passed through a filter to remove the formed precipitate and the polymeric adsorbent and wherein the precipitate and polymeric adsorbent freed supernatant is recovered in form of a flow-through.

Depending on the sample that is processed, the supernatant can be strongly coloured. Furthermore, certain samples such as certain food samples may also form more than one phase after sedimentation of the precipitate, what can be, as described, assisted by centrifugation. Sometimes, even three phases are formed after the precipitation step. In this case, the clear middle phase is separated as supernatant. Sometimes, the upper phase forms a semi-solid film (e.g. sometimes observed with specific sample types such as for example chocolate samples); this film can be pierced and only the clear middle phase is transferred as supernatant. These steps can be performed as it is well-known and described in the prior art.

The separation of the precipitate and collection of the supernatant is basically performed analogous to prior art methods and the skilled person is well familiar therewith. One difference to prior art methods is that the polymeric adsorbent that is additionally used in the present invention to improve the removal of inhibitors is also removed along with the precipitates.

After separation of the precipitate, a supernatant is obtained which contains the target nucleic acid, but which is due to the use of the at least one cationic detergent in combination with the at least one water-insoluble non-ionic polymeric adsorbent depleted of inhibitors that are contained in the sample. The target nucleic acid can then subsequently be isolated from the supernatant, if desired, or the obtained supernatant can be directly used for detection/analysis, depending on the used analytical method.

The extracted target nucleic acid, such as DNA, remains in solution and thus is contained in the supernatant. According to one embodiment, the obtained inhibitor-depleted supernatant is further processed in order to isolate the contained target nucleic acid therefrom. These subsequent nucleic acid isolation steps are described in the following.

### Steps c) and d)

Preferably, the method of the first aspect according to the invention comprises the following further steps:
c) optionally performing a further extraction step with the obtained supernatant using at least one organic solvent;
d) isolating the nucleic acid from the supernatant or the aqueous phase obtained after optional extraction step c).

According to one embodiment, at least one additional extraction step is performed with the obtained supernatant. For the additional extraction, also only a portion of the obtained supernatant can be used. For this purpose, an organic solvent can be used that is not miscible with water. Examples include but are not limited to chloroform, brome-chloropropane, hexane, diethylether, mixtures of phenol/chloroform and the like. Suitable organic solvents are known to the skilled person. Also, more than one organic solvent can be used in this step. This further extraction step using an organic solvent that is not miscible with water ensures that any remaining cationic detergent-protein, cationic detergent-debris, or cationic detergent-polysaccharide complexes that were not efficiently precipitated and/or separated in step b) is removed along with other lipophilic inhibitors into the organic phase. In case a respective further organic extraction step c) is performed, the aqueous phase containing the target nucleic acid is then processed further in order to isolate the target nucleic acid therefrom. This likewise encompasses using a portion of the supernatant in order to isolate the target nucleic acids therefrom.

In step d), the target nucleic acid is isolated from the supernatant obtained in step b) or the aqueous phase that is obtained after optional extraction step c). For this purpose, any suitable target nucleic acid isolation protocol can be used and the skilled person is familiar with respective methods. Exemplary embodiments are described herein.

For isolating one or more target nucleic acids (e.g. DNA and/or RNA) from the obtained supernatant, respectively the aqueous phase, methods known in the prior art may be used. Examples of suitable isolation methods include but are not limited to solid-phase extraction, silica-based purification methods, nucleic acid isolation procedures using chaotropic agents and/or at least one alcohol and a nucleic acid binding solid phase, alcohol precipitation based methods, magnetic particle-based purification methods, chromatography based purification procedures, anion-exchange chromatography (using anion-exchange surfaces, such as columns or magnetic particles), precipitation and combinations thereof. Such methods are known in the prior art and thus, do not need a detailed description here. Preferably, one or more target nucleic acids such as DNA and/or RNA is isolated from the supernatant, respectively the aqueous phase, by binding the target nucleic acid to a solid phase using appropriate binding conditions. Suitable methods are well-known to the skilled person.

As nucleic acid binding solid phase that can be used for binding the target nucleic acid (e.g. DNA), any material that is capable of binding the target nucleic acid can be used. This includes a variety of materials capable of binding nucleic acids. Exemplary solid phases that can be used in conjunction with the present invention include, but are not limited to, compounds comprising silicon, including but not limited to, silica materials such as silica particles, silica fibres, silica membranes, glass fibres, silicon dioxide, diatomaceous earth, glass, alkylsilica, aluminum silicate, and borosilicate; nitrocellulose; diazotized paper; hydroxyapatite (also referred to as hydroxyl apatite); nylon; metal oxides; minerals, zirconia; alumina; polymeric supports, organic polymers, diethylaminoethyl- and triethylaminoethyl-derivatized supports, hydrophobic chromatography resins and the like. The term solid phase is not intended to imply any limitation regarding its form or design. Thus, the term solid phase encompasses appropriate materials that are porous or non-porous, permeable or impermeable, including but not limited to membranes, filters, sheets, particles, magnetic particles, beads, gels, powders, fibers and the like. According to one embodiment, a solid phase functionalized with anion exchange ligands is used in order to bind the nucleic acid of interest from the protein-depleted supernatant. E.g. a column or particles such as magnetic particles functionalized with anion exchange ligands may be used. According to another embodiment, the surface of the solid phase such as e.g. a silica solid phase is not modified and is, e.g., not modified with functional groups.

Particularly preferred is the use of silicon containing materials such as silica and polysilicic acid materials, borosilicates, silicates and anorganic glasses as solid phase. Here, the solid phase preferably provides a silica surface for interaction with the nucleic acids which may be bound by precipitation and/or adsorption. The term "silica surface" as used herein includes surfaces comprising or consisting of silicon dioxide and/or other silicon oxides, diatomaceous earth, silica silanes, glass, zeolithe, bentonite, alkylsilica, aluminum silicate and borosilicate. The silica surface is preferably unmodified. Therefore, the surface is not modified with nucleic acid binding ligands or other nucleic acid binding groups. E.g., the solid phase does not carry any ligands at its binding surface that comprise ion exchange groups, in particular, the surface of the solid phase is not modified with functional ligands. In particular, it is not modified with ligands comprising anionic or cationic exchange groups such as e.g. amine groups or carboxyl groups. According to one embodiment, the silica surface does not comprise any functional groups besides its silanol groups or other oxidized forms of silicon, like oxides. Exemplary solid phases that can be used in conjunction with the present invention include, but are not limited to, solid phases providing a silica surface, including but not limited to, silica particles, silica fibres, glass materials such as e.g. glass powder, glass fibres, glass particles or controlled pore glass, silicon dioxide, glass or silica in particulate form such as powder, beads or frits. According to the present invention, the use of a column based solid phase or the use of particles, in particular magnetic particles, is preferred. Silica based nucleic acid isolation methods are broadly used in the prior art for isolating nucleic acids such as DNA and work particularly well if the binding mixture contains at least one salt, such as a chaotropic salt and optionally an alcohol.

According to one embodiment, silica particles are used that may have the form of beads. Preferably, said particles have a size of about 0.02 to 30 µm, more preferred 0.05 to 15 µm and most preferred of 0.1 to 10 µm. To ease the processing of the nucleic acid binding solid phase, preferably magnetic silica particles may be used. Magnetic particles respond to a magnetic field. The magnetic silica particles may e.g. be ferrimagnetic, ferromagnetic, paramagnetic or superparamagnetic. Suitable magnetic silica particles are for example described in WO 01/71732, WO 2004/003231 and WO 2003/004150. Other magnetic silica particles are also known from the prior art and are e.g. described in WO 98/31840, WO 98/31461, EP 1 260 595, WO 96/41811 and EP 0 343 934 and also include for example magnetic silica glass particles. The use of magnetic particles is convenient, because the magnetic particles including the bound target nucleic acid can be processed easily by the aid of a magnetic field, e.g. by using a permanent magnet. This embodiment is compatible with established robotic systems capable of processing magnetic particles. Here, different robotic systems exist in the prior art that can be used in conjunction with the present invention to process the magnetic particles to which nucleic acids were bound. According to one embodiment, magnetic particles are collected at the bottom or the side of a reaction vessel and the remaining liquid sample is removed from the reaction vessel, leaving behind the collected magnetic particles to which the nucleic acids are bound. Removal of the remaining sample can occur by decantation or aspiration. Such systems are well known in the prior art and thus need no detailed description here. In an alternative system that is known for processing magnetic particles the magnet which is usually covered by a cover or envelope plunges into the reaction vessel to collect the magnetic particles. As respective systems are well-known in the prior art and are also commercially available (e.g. QIASYMPHONY®; QIAGEN), they do not need any detailed description here. In a further alternative system that is known for processing magnetic particles, the sample comprising the magnetic particles can be aspirated into a pipette tip and the magnetic particles can be collected in the pipette tip by applying a magnet e.g. to the side of the pipette tip. The remaining sample can then be released from the pipette tip while the collected magnet particles which carry the bound nucleic acids remain due to the magnet in the pipette tip. The collected magnetic particles can then be processed further. Such systems are also well-known in the prior art and are also commercially available (e.g. BioRobot EZ1, QIAGEN) and thus, do not need any detailed description here.

According to one embodiment, a column based nucleic acid isolation procedure is performed, wherein the solid phase is comprised in a column. The term "column" as used herein in particular describes a container having at least two openings. Thereby, a solution and/or sample can pass through said column. The term "column" in particular does not imply any restrictions with respect to the shape of the container which can be e.g. round or angular and preferably is cylindrical. However, also other shapes can be used, in particular when using multi-columns. The column comprises the solid phase that is used for nucleic acid binding. Said solid phase comprised in the column should allow the passage of a solution, respectively the binding mixture when applied to the column. This means that if e.g. a centrifuge force is applied to the column, a solution and/or the binding mixture is enabled to pass through the column in direction of the centrifuge force. When using a respective column based nucleic acid isolation procedure, the binding mixture is usually passed through the column, e.g. assisted by centrifugation or vacuum, and the nucleic acid molecules bind to the comprised solid phase during said passage. The column can be used in a single format or in a multi-format. Such multi-columns having a similar format as multi-well plates and which comprise a solid phase such as a silica membrane or glass fibres, are well-known in the prior art and are also commercially available. Preferably, the column is a spin column. Preferably, a nucleic acid binding membrane or nucleic acid binding fibres are used as solid phase. Examples include but are not limited to silica membranes, glass fibre membranes or filters providing a silicon containing surface for nucleic acid binding. Preferably, the membrane is porous. As is shown by the examples, using a solid phase comprised in a column has several advantages. The use of columns such as spin columns is widely established for nucleic acid purification, and thus, the use of columns is very convenient for the user. Column based methods are also fast and, furthermore, automated systems exist that allow the automated processing of the samples (see e.g. QIAcube, QIAGEN). Thereby, tedious manual handling procedures can be avoided. Furthermore, using a spin column based approach for isolating a target nucleic acid has the advantage that there is no risk of carryover of potentially inhibitory components from the washing solutions (such as e.g. alcohol) or beads. It is preferred to use a membrane or fibres as solid phase which comprise or consist of silica in the column. Suitable and preferred silica based materials which provide a silica surface suitable for nucleic acid binding were also described above. A further common solid phase comprised in a column is a fill of silica particles, or a layer of a silica material (e.g. a silica gel). E.g. the silica particles can be arranged as a layer on an inert filter or membrane, thereby forming a nucleic acid binding solid phase. To alleviate the passage of the binding mixture through the solid phase comprised in the column, suitable means can be used such as e.g. centrifugation or the use of a pressure difference-generating apparatus which e.g. presses the sample through the column, respectively the solid phase or sucks it through the solid phase by applying a vacuum. Respective means are well known in the prior art and thus need no further description here.

The above described nucleic acid binding solid phases are generally suitable for binding DNA as well as RNA depending on the used binding conditions as is known to the skilled person.

According to one embodiment, the binding of the nucleic acids to the solid phase is performed in step d) in the presence of at least one salt, preferably a chaotropic salt and/or in the presence of at least one water-miscible organic solvent, such as an alcohol. Also a mixture of salts such as chaotropic salts can be used. Preferably, the salt such as a chaotropic salt is added in form of a binding solution. The concentration of the salt or mixture of salts in the binding solution may lie in a range of 0.05 M up to the saturation limit. Suitable concentration ranges lie, depending on the salt(s) used, within 0.1 M to 7 M, 1 M to 7 M, 1.5 M to 6 M and 2 M to 4 M. The described concentrations are particularly preferred for chaotropic salts. A higher salt concentration in the binding mixture is preferred in order to also capture small target nucleic acids or nucleic acid fragments. These principles are known in the art. The concentration of the salt in the binding mixture can be adjusted according to the needs e.g. by varying the volume of the binding solution compared to the volume of supernatant or aqueous phase that is processed.

Suitable chaotropic salts that can be used to promote binding of the nucleic acids to the solid phase all well-known in the art and include but are not limited to guanidinium hydrochloride, guanidinium thiocyanate, guanidinium isothiocyanate, sodium thiocyanate, sodium iodide, sodium perchlorate, sodium trichloroacetate, sodium trifluoroacetate, urea and the like and in particular preferred are guanidinium hydrochloride, guanidinium thiocyanate and guanidinium isothiocyanate.

As alcohol that can be used to promote nucleic acid binding, it is preferred to use one or more short chained branched or unbranched alcohols with preferably 1 to 5 carbon atoms. Examples are methanol, ethanol, propanol, isopropanol and butanol. Also mixtures of alcohols can be used. The alcohol is preferably selected from isopropanol and ethanol, particularly well suitable is isopropanol. The alcohol may be comprised in the binding mixture or in the binding solution in a concentration selected from 10 % v/v to 90 % v/v, 12.5 % v/v to 70 % v/v, 15 % to 60 % v/v and 17.5 % v/v to 50 %v/v. Suitable alcohol concentrations for binding large and small nucleic acids are also known in the art.

The term "binding mixture" as used herein in particular refers to the composition wherein the binding conditions were established for binding the nucleic acids such as in particular DNA to a nucleic acid binding solid phase.

After binding, the bound target nucleic acid can be washed. For this purpose common washing solutions may be used. According to one embodiment, the solution used for washing comprises at least one chaotropic agent and/or at least one alcohol. Chaotropic agents that can be used in the washing solutions include but are not limited to chaotropic salts such as guanidinium hydrochloride, guanidinium thiocyanate, guanidinium isothiocyanate and sodium iodide. Other chaotropic salts are also described above and can be used in the washing buffer. As alcohol, short chained branched or unbranched alcohols with preferably 1 to 5 carbon atoms can be used for washing, respectively in the washing solution. Examples are methanol, ethanol, propanol, isopropanol and butanol. Preferably, isopropanol and/or ethanol are used. However, also washing solutions without a chaotropic agent can be used.

For many downstream applications, it is possible to analyse, e.g. detect, the target nucleic acids while they are bound to the solid phase. Thus, according to one embodiment, the solid phase is directly subjected with the bound nucleic acids to an analytical method such as e.g. an amplification reaction. It is well known that it is e.g. possible to directly subject particles or another type of solid phase with the bound nucleic acids into a PCR reaction. The nucleic acids are here at least partially eluted due to the PCR conditions.

However, according to one embodiment, a separate elution step is performed in step d) to elute the nucleic acids from the solid phase.

In case it is desired to perform an elution step to elute the bound target nucleic acid from the solid phase, elution can be achieved for example with classical elution solutions such as water, elution buffers, in particular low salt elution buffers. The elution buffers may comprise a biological buffer such as Tris, MOPS, HEPES, MES, BIS-TRIS propane and others. If a salt is contained in the buffer, the salt concentration in the elution buffer is according to one embodiment ≤ 100 mM, ≤ 75 mM, ≤ 50 mM, ≤ 30 mM or ≤ 25 mM. A respective elution may be performed in step d). Preferably, elution solutions are used that do not interfere with the intended downstream applications. After elution, the eluate can be heat denatured. However, it is also within the scope of the present invention to release and thus elute the nucleic acids from the solid phase by other or supporting elution means such as e.g. heating.

### The sample and the target nucleic acids

The sample can be a biological or environmental sample such as a food, plant, animal, soil, microorganism or water sample. The method is particularly suitable for processing inhibitor rich samples. Typical inhibitors can be proteins, polysaccharides, polyphenols, fats, chemical flavor enhancers, antioxidants and salts. According to one embodiment, the sample is a food sample. Food samples may include but are not limited to raw food, processed food, cooked food, pasteurized food, dried food, meat, fish, poultry, vegetables, eggs, dairy products, such as cheese, milk, yoghurt, cream, bakery products, chocolate, peanut butter, ketchup, flour, cornflakes, beverages and the like. The method according to the present invention is in particular suitable for processing difficult samples that comprise a high concentration of inhibitors but only a low amount of nucleic acids. Advantageously, when using the method according to the present invention it is not necessary to distinguish between specific sample types, e.g. samples comprising a high or a low amount of inhibitors, because diverse samples can be efficiently processed with the method according to the present invention using a uniform protocol.

As nucleic acids, DNA and/or RNA can be isolated. The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. Nucleic acids include, but are not limited to all types of DNA and/or RNA, e.g. genomic DNA; circular DNA; plasmid DNA; mitochondrial DNA, circulating DNA; PNA; LNA, cyclohexene nucleic acids; RNA/DNA hybrids; hnRNA; mRNA; noncoding RNA (ncRNA), including but not limited to rRNA, tRNA, miRNA (micro RNA), siRNA (small interfering RNA), snoRNA (small nucleolar RNA), snRNA (small nuclear RNA), pwi-interacting RNA (piRNA), repeat associated RNA (rasiRNA), as RNA and stRNA (small temporal RNA); fragmented nucleic acids; synthetic nucleic acids, extracellular nucleic acids. The method according to the present invention is in particular suitable for isolating DNA, in particular genomic DNA from different samples, such as preferably from food samples. According to one embodiment, DNA and RNA is isolated as total nucleic acids from the sample.

### Further preferred embodiments

Particularly preferred embodiments are again described for illustration purposes subsequently. Details of the individual steps a) and b) and optional steps c) and d) as well as of the extraction mixture and the extraction composition that can be used to prepare the extraction mixture are also described above and it is referred to the respective disclosure which also applies here.

According to one embodiment, the method for extracting a target nucleic acid from a sample and depleting inhibitors contained in the sample comprises the following steps:
a) preparing an extraction mixture by contacting the sample with
   i) at least one cationic detergent capable of forming a precipitate with inhibitors contained in the sample, preferably CTAB,
   ii) at least one alkali metal halogenide salt, preferably sodium chloride or potassium chloride, wherein the salt is contained in the extraction mixture in a concentration to prevent precipitation of the target nucleic acid in the presence of the cationic detergent,
   iii) at least one water-insoluble non-ionic polymeric adsorbent, and
   iv) optionally, a proteolytic enzyme;
b) incubating the extraction mixture and forming a precipitate which comprises the cationic detergent and inhibitors contained in the sample and separating the formed precipitate and the polymeric adsorbent from the supernatant, thereby providing an inhibitor depleted supernatant which comprises the extracted target nucleic acid.
Said method may additionally comprise
c) optionally performing a further extraction step with the obtained supernatant using at least one organic solvent;
d) isolating the nucleic acid from the supernatant or the aqueous phase obtained after extraction step c).

According to one embodiment, the method for extracting a target nucleic acid from a sample and depleting inhibitors contained in the sample comprises the following steps:
a) preparing an extraction mixture by contacting the sample with
   i) at least one cationic detergent capable of forming a precipitate with inhibitors contained in the sample, preferably CTAB,
   ii) at least one salt, preferably an alkali metal halogenide, more preferably sodium chloride or potassium chloride, wherein the salt is contained in the extraction mixture in a concentration to prevent precipitation of the target nucleic acid in the presence of the cationic detergent,
   iii) at least one water-insoluble non-ionic polymeric adsorbent, and
   iv) optionally, a proteolytic enzyme;
      wherein for preparing the extraction mixture, the sample is contacted with an extraction composition which comprises the at least one salt and the at least one cationic detergent and wherein the salt of the extraction composition is present in the extraction mixture in a concentration that lies in the range of 0.5 M to 3M and wherein the cationic detergent of the extraction composition is present in the extraction mixture in a concentration that lies in the range of 0.7 to 5%;
b) incubating the extraction mixture and forming a precipitate which comprises the cationic detergent and inhibitors contained in the sample and separating the formed precipitate and the polymeric adsorbent from the supernatant, thereby providing an inhibitor depleted supernatant which comprises the extracted target nucleic acid.

Details regarding the extraction mixture and extraction composition, in particular suitable and preferred salt and cationic detergent concentrations, optional further additives and incubation conditions were described above and it is referred to the above disclosure which also applies here. Said method may additionally comprise
c) optionally performing a further extraction step with the obtained supernatant using at least one organic solvent;
d) isolating the nucleic acid from the supernatant or the aqueous phase obtained after extraction step c).

According to one embodiment, the method for extracting a target nucleic acid from a sample and depleting inhibitors contained in the sample comprises the following steps:
a) preparing an extraction mixture by contacting the sample with
   i) at least one cationic detergent capable of forming a precipitate with inhibitors contained in the sample, preferably CTAB,
   ii) at least one salt, preferably an alkali metal halogenide, more preferably sodium chloride or potassium chloride, wherein the salt is contained in the extraction mixture in a concentration to prevent precipitation of the target nucleic acid in the presence of the cationic detergent,
   iii) at least one water-insoluble non-ionic polymeric adsorbent, wherein optionally, the water-insoluble non-ionic polymeric adsorbent is selected from
      aa) a porous, cross-linked phenol-formaldehyde polymer having an average pore diameter in the range of 350 to 1,000 Angstrom, 400 to 800 Angstrom or 500 to 700 Angstrom and a BET surface in the range of 100 to 500 m²/g or 125 to 350 m²/g, and
      bb) a porous, aliphatic, acrylic ester polymer having an average pore diameter in the range of 50 to 300 Angstrom, 70 to 200 Angstrom or 75 to 150 Angstrom, a BET surface in the range of 200 to 700 m²/g, 250 to 650 m²/g or 300 to 550 m²/g and a dipole moment in the range of 1.5 to 2.0 or 1.6 to 1.9, and
   iv) optionally, a proteolytic enzyme;
   wherein for preparing the extraction mixture, the sample is contacted with an extraction composition which comprises the at least one salt and the at least one cationic detergent and wherein the salt of the extraction composition is present in the extraction mixture in a concentration that lies in the range of 0.55 M to 2.75 M, optionally in a range selected from 0.6 M to 2.5 M, 0.7 M to 2 M, 0.8 M to 1.75 M and 1.0 M to 1.5 M and wherein the cationic detergent of the extraction composition is present in the extraction mixture in a concentration that lies in the range of 0.85 to 4%, optionally in a range selected from 1% to 3.5%, 1.2% to 3%, 1.4% to 2.5%, 1.5% to 2.25% and 1.7 to 2%;
b) incubating the extraction mixture and forming a precipitate which comprises the cationic detergent and inhibitors contained in the sample and separating the formed precipitate and the polymeric adsorbent from the supernatant, thereby providing an inhibitor depleted supernatant which comprises the extracted target nucleic acid;
c) optionally performing a further extraction step with the obtained supernatant using at least one organic solvent;
d) isolating the nucleic acid from the supernatant or the aqueous phase obtained after optional extraction step c).

According to one embodiment, the method for extracting a target nucleic acid from a sample and depleting inhibitors contained in the sample comprises the following steps:
a) preparing an extraction mixture by contacting the sample with
   i) CTAB,
   ii) at least one alkali metal halogenide salt, preferably sodium chloride or potassium chloride, wherein the salt is contained in the extraction mixture in a concentration to prevent precipitation of the target nucleic acid in the presence of CTAB,
   iii) at least one water-insoluble non-ionic polymeric adsorbent, wherein the water-insoluble non-ionic polymeric adsorbent is selected from
      aa) a porous, cross-linked phenol-formaldehyde polymer having an average pore diameter in the range of 350 to 1,000 Angstrom, 400 to 800 Angstrom or 500 to 700 Angstrom and a BET surface in the range of 100 to 500 m²/g or 125 to 350 m²/g, and
      bb) a porous, aliphatic, acrylic ester polymer having an average pore diameter in the range of 50 to 300 Angstrom, 70 to 200 Angstrom or 75 to 150 Angstrom, a BET surface in the range of 200 to 700 m²/g, 250 to 650 m²/g or 300 to 550 m²/g and a dipole moment in the range of 1.5 to 2.0 or 1.6 to 1.9, and
   iv) a proteolytic enzyme;
   wherein for preparing the extraction mixture, the sample is contacted with an extraction composition which comprises the alkali metal halogenide salt and CTAB and wherein the salt of the extraction composition is present in the extraction mixture in a concentration that lies in the range of 0.6 M to 2.5 M and wherein CTAB is present in the extraction mixture in a concentration that lies in the range of 1% to 3.5%;
b) incubating the extraction mixture and forming a precipitate which comprises CTAB and inhibitors contained in the sample and separating the formed precipitate and the polymeric adsorbent from the supernatant, thereby providing an inhibitor depleted supernatant which comprises the extracted target nucleic acid;
c) optionally performing a further extraction step with the obtained supernatant using at least one organic solvent;
d) isolating the nucleic acid from the supernatant or the aqueous phase obtained after optional extraction step c).

The target nucleic acid is preferably DNA.

According to one embodiment, the method comprises
a) preparing an extraction mixture by contacting a preferably homogenized and/or disrupted food sample with
   i) CTAB,
   ii) at least one salt, wherein the salt is contained in the extraction mixture in a concentration to prevent precipitation of the target nucleic acid in the presence of CTAB,
   iii) at least one water-insoluble non-ionic polymeric adsorbent, and
   iv) a proteolytic enzyme;
b) incubating the extraction mixture at elevated temperature under agitation and forming a precipitate comprising CTAB and inhibitors that are contained in the food sample and separating the formed precipitate and the polymeric adsorbent from the supernatant, thereby providing an inhibitor depleted supernatant which comprises extracted DNA;
c) performing a further extraction step with the obtained supernatant using an organic solvent, preferably chloroform;
d) isolating DNA from the aqueous phase obtained from step c).

As described above, DNA may be isolated together with RNA.

The method according to the invention may furthermore comprise a step
e) analyzing the isolated target nucleic acid, preferably using an amplification based method.

In particular, such analysis may comprise the detection of the presence or absence of one or more nucleic acids such as DNA. The analysis may be performed for food authentication and/or the detection of GMO nucleic acids such as GMO DNA, in the isolated nucleic acids.

The analysis of the isolated nucleic acids can be performed using any nucleic acid analysis method including, but not limited to amplification technologies, polymerase chain reaction (PCR), isothermal amplification, reverse transcription polymerase chain reaction (RT-PCR), quantitative real time polymerase chain reaction (Q-PCR), digital PCR, gel electrophoresis, capillary electrophoresis, mass spectrometry, fluorescence detection, ultraviolet spectrometry, hybridization assays, DNA or RNA sequencing, restriction analysis, reverse transcription, NASBA, allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. Respective technologies are well-known to the skilled person and thus, do not need further description here. In particular preferred are methods that are based on nucleic acid amplification. Nucleic acids in foods, in particular processed foods, are often found at lower levels than in other biological samples. The nucleic acid extraction method described herein is very efficient and also allows to process large amounts of inhibitor rich samples. Purified nucleic acids are provided which can be subjected to an amplification based analysis because inhibitors are efficiently removed.

### KIT

Furthermore, a kit for extracting a nucleic acid from a sample is provided, comprising:
a) an extraction composition comprising
   i) at least one salt in a concentration selected from 0.7 M to 3 M, 0.8M to 2.75M, 0.9M to 2.5M, 1 M to 2 M, 1.1 M to 1.75M and 1.2 M to 1.5 M; and
   ii) at least one cationic detergent in a concentration selected from 1 to 5 %, 1.25 to 4%, 1.5% to 3.5%, 1.7% to 3%, 1.8% to 2.75%, 1.9% to 2.5% and 2.0 to 2.2 % wherein the cationic detergent is capable of forming a precipitate with inhibitors contained in the sample;
b) at least one water-insoluble non-ionic polymeric adsorbent and
c) optionally a proteolytic enzyme.

Such kit can be provided advantageously for use in automated systems. When using such kit, an inhibitor-depleted supernatant is provided from which the target nucleic acids can be isolated, e.g. by binding them to a solid phase. As described above, preferably magnetic particles, in particular magnetic silica particles, are used as nucleic acid binding solid phase. Details with respect to the extraction composition, the contained salt, the contained cationic detergent and the extraction conditions, the water-insoluble non-ionic polymeric adsorbent and the proteolytic enzyme were described in detail above and it is referred to the above disclosure which also applies here in conjunction with the kit. The kit can be used in the method according to the invention. It can be used for the isolation of DNA and RNA. It is particularly suitable for isolating DNA, in particular genomic DNA from samples, preferably food samples.

This invention is not limited by the exemplary methods and materials disclosed herein, and any methods and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

As used in the subject specification and claims, the singular forms "a", "an" and "the" include plural aspects unless the context clearly dictates otherwise. Thus, for example, reference to "a sodium salt" includes a single type of sodium salt, as well as two or more sodium salts. Likewise, reference to a "a cationic detergent", a "buffering agent", a "precipitate" and the like includes single entities and combinations of two or more of such entities. Reference to "the disclosure" and "the invention" and the like includes single or multiple aspects taught herein; and so forth. Aspects taught herein are encompassed by the term "invention".

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### EXAMPLES

### 1. Example 1

The DNeasy *mericon* Food kit (QIAGEN) was used as reference. Here, 10ml of a classical CTAB extraction buffer is used as food lysis buffer in combination with proteinase K in order to lyse the food sample (2g per preparation).

In brief, the DNeasy *mericon* Food kit protocol is performed as follows:
1. 2g of a homogenized food sample is placed in a 50 ml centrifuge tube and 10 ml CTAB food lysis buffer and 25 µl proteinase K solution is added. The mixture is vortexed briefly to ensure complete distribution and moistening of the sample material.
2. The so formed extraction mixture is incubated for 30 minutes at 60°C with constant shaking. To enhance inhibitor precipitation, the sample is cooled to room temperature (15 - 25°C) on ice after incubation.
3. The extraction mixture is centrifuged for 5 minutes at 2,500 x g in order to separate the precipitate from the supernatant.
4. 500 µl chloroform is added into a 2 ml micr centrifuge tube.
5. 700 µl of the clear supernatant from step 3 is transferred into the chloroform containing microcentrifuge tube.
6. The microcentrifuge tube is vortexed vigorously for 15 seconds and then centrifuged at 14,000 x g for 15 minutes in order to separate the phases.
7. 350 µl buffer PB (contains guanidine hydrochloride and isopropanol) is added into a fresh 2 ml microcentrifuge tube, and 350 µl of the upper, aqueous phase, from step 6 is added and mixed thoroughly by vortexing.
8. The composition from step 7 is added onto a QIAquick Spin column (comprising a silica membrane) for nucleic acid binding and centrifuged at 17,900 x g for one minute. The flow-through is discarded.
9. The bound nucleic acid is washed by adding 500 µl buffer AW2 (comprising ethanol) and centrifuged at 17,900 x g for one minute. The flow-through is discarded. The membrane is dried by an additional centrifugation step to remove remaining traces of ethanol.
10. 150 µl elution buffer EB is added directly onto the QIAquick membrane. After incubation for one minute at room temperature, the spin column is centrifuged at 17,900 x g for one minute to elute the bound nucleic acid.

The eluate containing the isolated nucleic acids is then ready for analysis.

This standard protocol was modified in the CTAB titration protocol in order to simulate a higher food content per preparation and to achieve a higher amount of processed DNA. The amount of sample remained the same but the usual CTAB buffer volume (10 ml) applied to precipitate CTAB accessible inhibitors was down-titrated to 9, 8, 7, and 6 ml, and additionally the subsequently processed aliquot of the supernatant (700 µl) was increased to 1 ml. The effects of these modifications were analysed in order to determine the effect of a higher food (and thus inhibitor) content per preparation. Peanut butter was used as a test matrix (food sample), providing a readily homogenized but inhibitor containing sample type. A defined amount of peanut butter was processed according to the DNeasy *mericon* Food Standard Protocol, applying besides the standard method the above described CTAB titration protocol. The overall extracted plant DNA from peanut butter was analyzed, using a generic plant PCR assay.

Fig. 1 illustrates the PCR results for the different CTAB buffer volumes and hence, food contents. In addition to the undiluted eluate a 1:10 diluted eluate was also measured by default. In an uninhibited PCR reaction, a 1:10 dilution should lead to a 3.33 shift to higher Ct values because the nucleic acid concentration is reduced due to the dilution. Differences below this value indicate the presence of inhibitors in the isolated DNA which disturb the PCR reaction. Thus, in such a case, the dilution not only dilutes the nucleic acids (what is negative for the PCR because less template is available), but also dilutes the inhibitors (what improves the PCR). Therefore, dilution of the nucleic acids (less template) is compensated in such case to some extent by the dilution of the PCR inhibitors so that the difference in the Ct values between diluted and undiluted sample is less than 3.33. The differences between the undiluted and the 1:10 diluted eluates obtained with the different amounts of CTAB buffer are plotted in Fig. 2.

From Fig. 1 and 2 it can be seen that a reduction of the CTAB buffer volume (thereby relatively increasing the amount of food sample that is processed per preparation) initially slightly decreases the absolute Ct values of the undiluted samples (9 and 8 ml) and thus improves the PCR result, because of the higher amount of processed DNA. I.e. the DNA concentration in the eluate (template) is higher, because the sample was processed in a lower volume of extraction buffer and more precipitate-free supernatant was used for isolating the nucleic acids (see above). However, the Ct difference between the undiluted and the 1:10 diluted eluate decreases simultaneously while data scattering increases. This clearly indicates a strong carry-over of inhibitors from the sample preparation into the eluate, crossing a critical threshold between 7 and 6 ml CTAB extraction buffer. When using only 6 ml of CTAB buffer (instead of 10ml) the inhibitor concentration in the eluate is so high that the 1:10 dilution is even about 5 Cts better than the undiluted sample, even though the dilution contains less template DNA.

On the basis of these experiments an amount of 7 ml CTAB extraction buffer was used to simulate the higher amount of food sample per preparation. This adapted method provides inhibitor carry-over in a manageable range (Ct difference undiluted/1:10 diluted: 1.29), in which an improvement of inhibitor removal was expected to show a visible Ct improvement without being overlaid by an excessive inhibitor concentration.

Different water-insoluble additives were included in the extraction mixture in order to test their capability to improve inhibitor removal. Approx. 500-600 mg water-insoluble additive was added in addition to the 7ml CTAB extraction buffer. The tested additives and their chemical characteristics are listed in Table 1.

| **Compound** | **Chemical nature of compound** | **Matrix** | **Surface functionality** |
|---|---|---|---|
| **Duolite® XAD-761** | **Phenol-formaldehyde polymer** | **Non-ionic** | **Hydroxy-methyl functionalities** |
| Duolite® A-7 | Formaldehyd-N,N'-bis(2-aminoethyl)-1,2-ethane-diamine-phenol polymer | Weakly basic anion exchanger, hydrophilic | Amine functionalities |
| Duolite® ES-468 | Acrylic acid polymer | Weakly acidic | |
| **Amberlite™ XAD-7** | **Aliphatic-acrylic polymer** | **Weakly polar, non-ionic** | **Acrylic ester functionalities** |
| Amberlite™ MB-1 | Styrene-divinylbenzene gel | Mixed cationic (strongly acidic) and anionic (strongly basic) exchange resin | Sulfonic acid and quaternary ammonium functionalities |
| Dowex™ Marathon A | Styrene-divinylbenzene copolymer | Strongly basic anion exchanger | Quaternary ammonium functionalities |
| QIAresin | Silane-coupled diethylamine | Weakly basic anion exchanger | Secondary amine functionalities |

Test materials thus included non-ionic adsorptive materials (Duolite® XAD-761 and Amberlite™ XAD-7 - marked in bold in Table 1), weakly basic or acidic ion exchangers (Duolite® A-7, Duolite® ES-468 and QIAresin) and strongly basic or mixed strongly basic & acidic styrene based ion exchangers (Amberlite™ MB-1 and Dowex™ Marathon A). According to the different chemical nature of the inhibitors such as charge, hydrophobicity or hydrophilicity, and size, these materials may individually interact with inhibitors of the tested food types, according to charge or dipol interactions, van der Waals driven adsorption or diffusion into macroporous resin structures. These water-insoluble additives are separable from the extraction mixture via centrifugation together with the precipitate so that no soluble excess material can interact and in particular interfere with the subsequent DNA isolation.

Otherwise, the samples were processed as described above, wherein, however, in contrast to the standard protocol, 1 ml precipitate freed supernatant was subjected to chloroform extraction. The water-insoluble additives were efficiently separated together with the CTAB precipitated inhibitors during the centrifugation step. Eluates were analyzed in a PCR reaction using the generic plant assay.

Fig. 3 illustrates the resulting Ct values of the undiluted and 1:10 diluted eluates obtained from the DNA extractions (7 ml CTAB buffer) of a peanut butter food matrix. Next to the above described water-insoluble additives, an extraction buffer supplemented with PVP was measured as a reference. PVP is a water soluble polymer already known as a functional inhibitor removal additive in DNA extraction applications. Due to its solubility PVP was applied to evaluate the influence of a permanent workflow additive. The additionally measured reference eluate was obtained from a separate prep experiment applying the unchanged DNeasy *mericon* Food Standard Protocol (10 ml CTAB buffer, 700µl supernatant) without any additive. In Fig. 3 it can be seen based on the high Ct values, that PVP is not an applicable compound for inhibitor removal when intending to process a higher amount of food sample. In case of PVP, co-processing of an inseparable compound excess throughout DNA purification might lead eventually to DNA scavenging. Compared to the reference eluate also the strongly basic and mixed strongly basic and acidic ion exchangers Amberlite™ MB-1 and Dowex^{™} Marathon A lead to a Ct increase of about 2 and about 3, respectively. This is most likely due to DNA binding of all three materials types. In case of the strongly acidic or basic exchanger materials strong charge interactions between the functional exchanger groups and e.g. the DNA phosphate groups (ion exchanger interactions) might be dominant compared to somewhat weaker inhibitor charge interactions. Also the Duolite® A-7 and Duolite® ES-468, weakly basic and weakly acid exchanger materials, are not applicable as inhibitor removal additives. Duolite® A-7 shows virtually no difference between the Cts of the undiluted and 1:10 diluted eluates (see Fig. 4), indicating a clear lack of inhibitor removal capacities. Although less pronounced, Duolite® ES-468 also shows only limited inhibitor removal efficiency (ΔCt ∼1.2).

In contrast to the listed weakly or strongly ionic exchanger materials, the tested non-ionic polymeric adsorbents showed good results and improved inhibitor depletion. Duolite® XAD-761 and Amberlite™ XAD-7 showed comparable to slightly better Ct values of the undiluted eluate compared to the measured reference eluate. Yet, the Ct difference between their undiluted and 1:10 diluted eluates already exceeds the difference of the reference eluate, thereby showing a significantly higher degree of inhibitor depletion despite that in the experimental set up, an initially higher amount of food and a more concentrated inhibitor background compared to the reference eluate were present. Therefore, the use of a non-ionic polymeric adsorbent greatly improved the depletion of inhibitors, thereby allowing to increase the amount of input sample and/or to lower the amount of extraction buffer per preparation. This is a significant advantage.

To verify that the non-ionic polymeric adsorbent interacts exclusively with the inhibitors to be depleted under the given reaction conditions, different materials were tested with an orange juice matrix. This food matrix has a high DNA content with a very low inhibitor concentration. It was analysed whether an unsaturated inhibitor scavenging material will eventually start to bind target DNA that is liberated during the lysis reaction. Compared to a 7 ml reference reaction without an additive, the tested non-ionic exchanger Duolite® XAD-761 showed no or only very little shifts to higher Ct values, thereby demonstrating that substantially no DNA binding occurred. Therefore, the polymeric adsorbents used according to the invention to assist the depletion of inhibitors, do not interfere with the nucleic acid isolation and hence, do not reduce the yield.

Overall it was shown that from the range of tested additives, charged compounds such as strongly or weakly basic/acidic exchanger materials give unfavorable results. It is likely that the desired charge attraction between the exchanger material and the inhibitor is partially compensated by equally strong charge repulsion processes. On the other hand, non-ionic polymeric adsorbents, like Duolite® XAD-761 and Amberlite™ XAD-7 showed very good results. These adsorbents interact with different inhibitors on the basis of their respective electron distribution, electron density or electron exchange reactions. This mode of interaction apparently results in a high binding selectivity of the non-ionic polymeric adsorbents for matrix inhibitors, while detrimental reactions with the target DNA could not be observed. These materials are therefore highly useful as functional inhibitor scavengers to enhance inhibitor depletion during nucleic acid extraction from inhibitor rich sample materials, such as processed food types.

## Claims

1. A method for extracting a target nucleic acid from a sample and depleting inhibitors contained in the sample, comprising:
a) preparing an extraction mixture by contacting the sample with
i) at least one cationic detergent capable of forming a precipitate with inhibitors contained in the sample,
ii) at least one salt, wherein the salt is contained in the extraction mixture in a concentration to prevent precipitation of the target nucleic acid in the presence of the cationic detergent, and
iii) at least one water-insoluble non-ionic polymeric adsorbent,
b) incubating the extraction mixture and forming a precipitate which comprises the cationic detergent and inhibitors contained in the sample and separating the formed precipitate and the polymeric adsorbent from the supernatant, thereby providing an inhibitor depleted supernatant which comprises the extracted target nucleic acid.

2. The method according to claim 1, wherein the polymeric adsorbent has one or more of the following characteristics:
aa) it is a resin, preferably a porous, spherical resin;
bb) it has a hydrophilic surface;
cc) it comprises one or more surface functionalities selected from acryl, acrylic ester, phenol, phenolic hydroxyl and/or methylol groups and hydroxyl-methyl functionalities;
dd) it has a dipole moment that lies within a range selected from of 0.5 to 2, 0.75 to 2, 1 to 2, 1.25 to 2, 1.5 to 2 and 1.6 to 1.9;
ee) it is a cross-linked polymer having a matrix that is based on phenol-formaldehyde, acrylate or polystyrene;
ff) it is porous;
gg) it is porous, wherein the average pore diameter lies within a range selected from 50 to 1,000 Angstrom, 60 to 750 Angstrom and 75 to 700 Angstrom and/or wherein the non-ionic polymeric adsorbent has a BET surface area (m²/g) that lies in a range selected from 100 to 1000 m²/g and 125 to 750 m²/g;
hh) it is a porous, cross-linked poly-phenolic polymer or a porous, aliphatic, acrylic ester polymer;
ii) it is selected from
i. a porous, cross-linked phenol-formaldehyde polymer having an average pore diameter in the range of 350 to 1,000 Angstrom, 400 to 800 Angstrom or 500 to 700 Angstrom and a BET surface in the range of 100 to 500 m²/g or 125 to 350 m²/g, and
ii. a porous, aliphatic, acrylic ester polymer having an average pore diameter in the range of 50 to 300 Angstrom, 70 to 200 Angstrom or 75 to 150 Angstrom, a BET surface in the range of 200 to 700 m²/g, 250 to 650 m²/g or 300 to 550 m²/g and a dipole moment in the range of 1.5 to 2.0 or 1.6 to 1.9;
and/or
jj) it is provided in the form of particles, wherein optionally, the particles have a mean diameter that lies in a range selected from 100 to 2000µm, 150 to 1750µm, 200 to 1500µm, 300 to 1250µm and 400 to 1000µm.

3. The method according to one or more of claims 1 or 2, having one or more of the following characteristics:
i) the salt is a non-chaotropic salt;
ii) the salt is an alkali metal salt, preferably an alkali metal halogenide, more preferably an alkali metal halogenide selected from sodium and potassium chloride;
iii) the extraction mixture comprises a proteolytic enzyme; and/or
iv) the extraction mixture has a pH selected from 7 to 9, 7.25 to 7.25 to 8.75, 7.5 to 8.5, 7.75 to 8.25 and 7.9 to 8.1.

4. The method according to one or more of claims 1 to 3, wherein in step a) the sample is contacted with an extraction composition comprising the at least one salt and the at least one cationic detergent.

5. The method according to claim 4, wherein the extraction composition has one or more of the following characteristics:
i) the extraction composition comprises the salt in a concentration selected from 0.7 M to 3 M, 0.8M to 2.75M, 0.9M to 2.5M, 1 M to 2 M, 1.1 M to 1.75M and 1.2 M to 1.5 M;
ii) the extraction composition comprises the cationic detergent in a concentration selected from 1 to 5 %, 1.25 to 4%, 1.5% to 3.5%, 1.7% to 3%, 1.8% to 2.75%, 1.9% to 2.5% and 2.0 to 2.2 %.

6. The method according to claim 5, wherein the extraction composition has one or more of the following characteristics:
i) the extraction composition comprises a buffering agent; and/or
ii) the extraction composition comprises a chelating agent optionally in a concentration selected from 10 mM to 50 mM, 10 mM to 35 mM, 15 mM to 30 mM, 18mM to 25 mM and 19 mM to 23 mM.

7. The method according to one or more of claims 1 to 6, wherein the cationic detergent has one or more of the following characteristics
i) it is a quaternary ammonium salt;
ii) it is a quaternary ammonium salt comprising at least one alkyl group having a chain length of from 6 to 22 carbon atoms, 8 to 20 carbon atoms, 10 to 18 carbon atoms, 12 to 18 carbon atoms or 16 carbon atoms;
iii) it is selected from the group consisting of cetyl trimethyl ammonium bromide (CTAB), tetra decyl trimethyl ammonium bromide (TTAB) and dodecyl trimethyl ammonium bromide (DTAB) or the corresponding compounds comprising a chloride instead of the bromide;
iv) it is CTAB.

8. The method according to one or more of claims 1 to 7, comprising
c) optionally performing a further extraction step with the obtained supernatant using an organic solvent;
d) isolating the target nucleic acid from the supernatant or the aqueous phase obtained after optional extraction step c).

9. The method according to claim 8, comprising
e) detecting the isolated target nucleic acid using an amplification based detection method.

10. The method according to one or more of claims 1 to 9, having one or more of the following characteristics:
a. the target nucleic acid is DNA;
b. DNA and RNA is isolated as target nucleic acid;
c. step b) has one or more of the following characteristics:
aa) the extraction mixture comprises a proteolytic enzyme and is incubated, optionally for 15 min to 60 min, preferably 20 min to 45 min;
bb) the extraction mixture is incubated at an elevated temperature, preferably at a temperature that lies in a range of 45 to 70°C, preferably 50 to 65°C,
cc) the extraction mixture is agitated during incubation; and/or
dd) the extraction mixture is incubated at elevated temperature and afterwards is cooled.

11. The method according to one or more of claims 1 to 10, wherein the sample is a food sample.

12. The method according to claim 11, comprising
a) preparing an extraction mixture by contacting a preferably homogenized food sample with
i) at least one salt;
ii) CTAB,
iii) at least one water-insoluble non-ionic polymeric adsorbent; and
iv) a proteolytic enzyme;
b) incubating the extraction mixture at elevated temperature and forming a precipitate comprising CTAB and inhibitors contained in the food sample and separating the formed precipitate and the non-ionic polymeric adsorbent from the supernatant, thereby providing an inhibitor depleted supernatant which comprises extracted DNA;
c) performing a further extraction step with the obtained supernatant using an organic solvent, preferably chloroform;
d) isolating DNA from the aqueous phase obtained from step c).

13. The method according to one or more of claims 8 to 12, wherein nucleic acid isolation in step d) involves binding the nucleic acid to a solid phase in the presence of a chaotropic salt and/or alcohol.

14. A kit for extracting a nucleic acid from a sample, comprising
a) an extraction composition comprising
i) at least one salt in a concentration selected from 0.7 M to 3 M, 0.8M to 2.75M, 0.9M to 2.5M, 1 M to 2 M, 1.1 M to 1.75M and 1.2 M to 1.5 M; and
ii) at least one cationic detergent in a concentration selected from 1 to 5 %, 1.25 to 4%, 1.5% to 3.5%, 1.7% to 3%, 1.8% to 2.75%, 1.9% to 2.5% and 2.0 to 2.2 %, wherein the cationic detergent is capable of forming a precipitate with inhibitors contained in the sample;
b) at least one water-insoluble non-ionic polymeric adsorbent; and
c) optionally a proteolytic enzyme.

15. The kit according to claim 14, comprising:
a) an extraction composition which is an aqueous composition comprising
i) at least one salt, preferably a sodium salt, in a concentration selected from 1 M to 2 M, 1.1 M to 1.75M or 1.2 M to 1.5 M;
ii) at least one cationic detergent, preferably CTAB, in a concentration from 1.7% to 3%, 1.8% to 2.75% or 1.9% to 2.25%;
iii) a buffering agent; and
iv) a chelating agent;
b) at least one water-insoluble non-ionic polymeric adsorbent, wherein preferably, the polymeric adsorbent has one or more of the characteristics as defined in claim 2.
